# EUROPÄISCHE PATENTSCHRIFT

(11) **EP 0 541 645 B1**
(45) Veröffentlichungstag und Bekanntmachung des Hinweises auf die Patenterteilung: **19.11.1997**
(21) Anmeldenummer: 91914044.2
(22) Anmeldetag: 26.07.1991
(51) Int. Cl.: C07K 1/16, C07K 16/30, G01N 33/566, C12P 21/08

(54) **VERFAHREN ZUR REINIGUNG VON CYTOKERATIN 20 UND DESSEN VERWENDUNG ZUR ERZEUGUNG VON ANTIKÖRPERN**
METHOD OF PURIFYING CYTOKERATIN 20, AND THE USE OF CYTOKERATIN 20 TO PRODUCE ANTIBODIES
PROCEDE DE PURIFICATION DE LA CYTOKERATINE 20 ET SON UTILISATION POUR PRODUIRE DES ANTICORPS

(30) Priorität: 27.07.1990 DE 4023945
(43) Veröffentlichungstag der Anmeldung: 19.05.1993
(73) Patentinhaber: PROGEN Biotechnik GmbH, 69120 Heidelberg (DE)
(72) Erfinder: MOLL, Roland, D-6900 Heidelberg (DE); FRANKE, Werner, Wilhelm, D-6900 Heidelberg (DE)
(74) Vertreter: Huber, Bernhard, Dipl.-Chem.
(86) Internationale Anmeldenummer: EP9101407
(87) Internationale Veröffentlichungsnummer: WO9202558

(56) Entgegenhaltungen:
- EP-A- 267 356
- EP-A- 337 057
- WO-A-85/03132
- DIALOG INFORMATION SERVICES, File 154, Medline 85-91; R. MOLL et al., Dialog AN 07431111
- DIALOG INFORMATION SERVICES, File 155, Medline 67-91; W.W. FRANKE et al., Dialog AN 05146776
- DIALOG INFORMATION SERVICES, File 155, Medline 67-91; R.A. QUINLAN et al., Dialog AN 05417706
- AMERICAN JOURNAL OF PATHOLOGY, vol. 136, no. 2, February 1990; H.E. SCHAAFSMA et al., pp. 329-343

## Beschreibung

Die Erfindung betrifft ein Verfahren zur Reinigung von Cytokeratin 20, ein Standardproteinmaterial sowie ein Verfahren zu seiner Herstellung, sowie ein Verfahren zur Herstellung von gegen CK 20 gerichteten Antikörpern und die Verwendung eines Antikörpers, der für CK 20 spezifisch ist zum Nachweis dieses Proteins auf Gewebeschnitten, in Gewebehomogenaten unc Körperflüssigkeiten, sowie die Verwendung eines Standardproteinmaterials zum Nachweis von Autoantikörpern gegen CK 20 im Blut oder Serum.

Es wurde festgestellt, daß die Intermediär-Filament (IF)-Proteine der Cytokeratin-Familie wirksame Marker für die Analyse der Art und des Differenzierungszustands von Epithelzellen sind. Die epithelialen Cytokeratine, umfassend eine Familie von mindestens 19 verschiedenen Polypeptiden, werden in verschiedenen Kombinationen, abhängig vom Verlauf der Zelldifferenzierung, exprimiert. Die Synthese von Cytokeratinen wird im allgemeinen aufrechterhalten während der malignen Transformation, und diese Tatsache könnte als eines der Nachweiskriterien von Epithel-abgeleiteten Tumoren, einschließlich Tumoren des Blasentraktes, dienen. Es bestand u.a. ein Bedürfnis nach einer leicht durchführbaren und sicheren Nachweismethode zur Feststellung der Lokalisierung des Primärtumors von Metastasengewebe, so daß gegen diesen Primärtumor effektiv vorgegangen werden kann. Der Erfindung lag daher die Aufgabe zugrunde, eine Möglichkeit bereitzustellen, die Unterscheidung verschiedener Tumorzellarten ebenso wie den Herkunftsnachweis für verschiedene Metastasengewebe leicht und möglichst exakt führen zu können.

Es wurde nun ein neues Cytokeratin identifiziert, das nur in bestimmten Zellen vorkommt und damit als Marker zur Unterscheidung bestimmter Zellen und Gewebe dienen kann. Es wurde Cytokeratin 20 genannt.

Ein Gegenstand der Erfindung ist daher ein Verfahren zur Reinigung von Cytokeratin 20, bei dem man eine Cytoskelettfraktion aus CK 20 enthaltenden Geweben oder Zellen herstellt, die darin enthaltenen Proteine gelelektrophoretisch oder/und chromatographisch auftrennt und das CK 20 aus dem Gel bzw. der das CK 20 enthaltenden Chromatographiefraktion gewinnt.

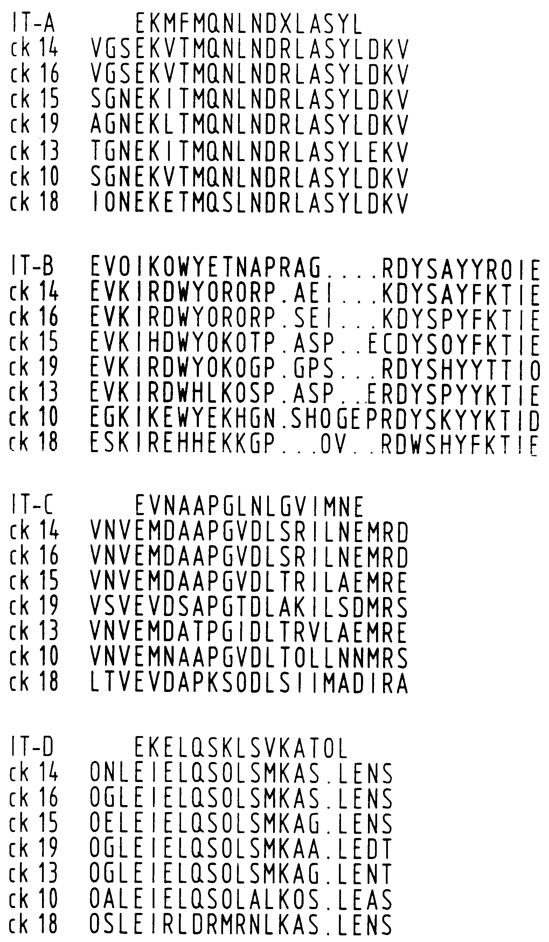

Das Protein CK 20 hat ein Molekulargewicht von ca. 46.000, einen isoelektrischen Punkt in 9,5 mol Harnstoff von ∼ 6,1 und ist etwas saurer als die nicht-phosphorylierte Variante von CK 8. Fig. 1 zeigt eine partielle Aminosäuresequenz von CK 20 (dort bezeichnet IT; A-D erhalten aus Fragmenten von CK 20 nach Verdauung mit Staphylococcus V8 Protease).

Erfindungsgemäß ist es möglich, das Cytokeratin 20 in einer so reinen Form zu erhalten, daß damit z.B. spezifische Antikörper hergestellt werden können. In einer Ausführungsform der Erfindung stellt man die Cytoskelettfraktion aus Duodenalschleimhautzotten, insbesondere von menschlichem Gewebe her. In einer anderen Ausführungsform der Erfindung gewinnt man die Cytoskelettfraktion aus Kulturzellen, wobei Kulturzellen bevorzugt sind, die aus Kolonkarzinomen, Blasenkarzinomen oder Magenkarzinomen abgeleitet sind. Bei der Durchführung einer Gelelektrophorese wird in einer bevorzugten Ausführungsform der Erfindung eine erste SDS-Polyacrylamidgelelektrophorese in einem Puffersystem mit erhöhter Salzkonzentration durchgeführt, danach wird die CK 20 enthaltende Bande ausgeschnitten und das Protein daraus eluiert und eine zweite Polyacrylamidgelelektrophorese in einem Puffersystem mit niedriger Salzkonzentration durchgeführt und aus der entsprechenden Bande im Gel das nunmehr gereinigte CK 20 isoliert.

In einer weiteren Ausführungsform der Erfindung, nämlich der auftrennung auf chromatographischem Wege, führt man zuerst eine Anionenaustauschchromatographie und dann eine Phasenumkehr-HPLC-Chromatographie durch. Hierbei ist es wiederum bevorzugt, die Anionenaustauschchromatographie an DEAE-cellulose in Anwesenheit von Harnstoff und unter Verwendung eines Eluens mit linearem Gradienten von zwischen 0 und 100 mmol/l Guanidiniumhydrochlorid durchzuführen und die CK 20 enthaltenden Fraktionen in der Folge der HPLC zu unterwerfen. Vorzugsweise unterwirft man dabei die Fraktionen, enthaltend 38 bis 50 mmol/l Guanidiniumhydrochlorid, der HPLC.

Ein weiterer Gegenstand der Erfindung ist ein Standardproteinmaterial, das aus einem rekonstituierten Cytokeratinkomplex, enthaltend CK 20 und ein basisches Cytokeratin aus der Gruppe der Cytokeratine 1 bis 8, oder aus den durch proteolytische Spaltung hergestellten entsprechenden α-helikalen Mittelstücken dieser Proteine besteht. Hierbei ist es bevorzugt, daß das Standardproteinmaterial aus einem Komplex, enthaltend CK 20 und CK 8, bzw. deren α-helikale Mittelstücke, besteht.

Ein derartiges Standardproteinmaterial ist ein geeignetes Mittel zum Einsatz in immunologischen Tests, beispielsweise unter Anwendung des Verdrängungsprinzipes oder sonstiger konkurrierender Immunoassays durchzuführen.

Ein weiterer Gegenstand der Erfindung ist auch ein Verfahren zur Herstellung eines Standardproteinmaterials, enthaltend CK 20 und ein basisches Cytokeratin, bei dem man gereinigtes CK 20 und ein gereinigtes basisches Cytokeratin aus der Gruppe der Cytokeratine 1 bis 8 im equimolaren Verhältnis gemeinsam in einem harnstoffhaltigen Puffer löst und die Mischung zuerst gegen einen Harnstoff und DTT enthaltenden Puffer, dann gegen einen Puffer ohne Harnstoff dialysiert. Durch dieses Verfahren wird ein rekonstituiertes Filamentmaterial erhalten, das sich bildet bei der Entfernung von Harnstoff aus dem Puffer. In einer bevorzugten Ausführungsform der Erfindung spaltet man nachfolgend aer Herstellung des Standardproteinmaterials den gebildeten Cytokeratinkomplex proteolytisch. Hierdurch werden die α-helikalen Mittelstücke des rekonstituierten Standardproteinmaterials erhalten. Diese proteolytische Spaltung wird vorzugsweise mit Chymotrypsin und in einem Enzym zu Substratverhältnis von 6:1000 bis 10:1000 und mit Verdauungszeiten zwischen 30 und 60 Minuten durchgeführt.

Erfindungsgemäß ist es besonders bevorzugt, als basiscnes Cytokeratin bei der Herstellung des Standardproteinmaterials CK 8 zu verwenden. Wiederum bevorzugt ist es, ein gereinigtes CK 20 zu verwenden, das nach dem erfindungsgemäßen Verfahren gereinigt wurde. In wiederum einer bevorzugten Ausführungsform der Erfindung löst man zur Herstellung eines Standardproteinmaterials die Proteine in einem 8,5 bis 10 mol/l Harnstoff und 1,5 bis 3 mmol/l DTT enthaltenden Puffer und führt die erste Dialyse gegen einen 3,5 bis 4,5 mol/l Harnstoff und 1,5 bis 3 mmol/l DTT enthaltenden Puffer durch.

Ein wiederum weiterer Gegenstand der Erfindung ist ein Verfahren zur Herstellung von für CK 20 spezifischen Antikörpern, bei dem man zur Immunisierung geeigneter Tiere gereinigtes CK 20 einsetzt und nach an sich bekannten Methoden dann polyklonale oder monoklonale Antikörper erzeugt. Die prinzipielle Erzeugung von polyklonalen Antikörpern ebenso wie von monoklonalen Antikörpern ist dem Fachmann bekannt und die Herstellung von monoklonalen Antikörpern beispielsweise von Köhler und Milstein in Nature 256 (1975) 495-497 beschrieben. Vorzugsweise werden mit dem gereinigten CK zur Gewinnung von polyklonalen Antikörpern Meerschweinchen immunisiert.

In einer bevorzugten Ausführungsform der Erfindung setzt man erfindungsgemäß gereinigtes CK 20 ein.

Um tatsächlich auch bei der Herstellung von polyklonalen Antikörpern monospezifiscne Antikörper, die gegen CK 20 gerichtet sind, zu erhalten, ist es wiederum erfindungsgemäß bevorzugt, zur Isolierung dieser monospezifischen Antikörper die Immunoglobulinfraktion einer Immunpräzipitation und Abtrennung von gegen andere Cytokeratine gerichteten Antikörpern oder/und einer Immunpräzipitation und Abtrennung der für CK 20 spezifischen Antikörper zu unterwerfen. Hierbei sind alle dem Fachmann bekannten Immunpräzipitationen geeignet, und es wird letztendlich ein Antikörper erhalten, der lediglich mit CK 20 immunologisch reagiert. In einer bevorzugten Ausführungsform der Erfindung führt man die Immunpräzipitation und Abtrennung der für CK 20 spezifischen Antikörper durch Inkubation der aus dem Versuchstier erhaltenen Immunglobulinfraktion mit einer festen Phase durch, an die CK 20 gekoppelt wurde. Bei der Immunpräzipitation und Abtrennung von gegen andere Cytokeratine gerichteten Antikörpern führt man vorzugsweise eine Inkubation der erhaltenen Immunglobulinfraktion mit einer festen Phase durch, an die elektrophoretisch aufgereinigte Cytokeratine 8, 18 und 19 oder Gesamtprotein aus diese enthaltenden Zellen gekoppelt wurde. Erfindungsgemäß werden die Immunpräzipitationsschritte vorzugsweise mehrfach durchgeführt, so daß auch tatsächlich sämtliche nicht gegen CK 20 gerichteten Antikörper von den monospezifiscnen Antikörpern abgetrennt werden. Erfindungsgemäß ist es wiederum bevorzugt, als feste Phase Nitrocellulosestreifen zu verwenden.

Ein weiterer Gegenstand der Erfindung ist die Verwendung eines Antikörpers, der gegen CK 20 gerichtet ist, zur immunologischen Identifizierung von CK 20 oder seinem durch proteolytische Spaltung erhaltenen α-helikalen Mittelstück auf GeweDeschnitten, in Gewebehomogenaten und in Körperflüssigkeiten. Hierbei ist es wiederum bevorzugt, aus einer GewebeproDe ein Homogenat zu bilden, die in dem Homogenat enthaltenen Intermediär-Filamentproteine proteolytisch zu spalten und die caraus freigesetzten α-helikalen Mittelstücke in der löslichen Phase abzutrennen und mit Hilfe des Antikörpers zu identifizieren und quantitativ zu bestimmen.

Eine wiederum bevorzugte Ausführungsform der Erfindung ermöglicht es, daß in Körperflüssigkeiten wie Blut, Blutserum und Urin die darin enthaltenen löslichen Intermediär-Filamentproteinfragmente mit Hilfe des Antikörpers immunologisch identifiziert und quantitativ bestimmt werden.

Durch die erfindungsgemäße Verwendung des Antikörpers gegen CK 20 wird es ermöglicht, festzustellen, ob dieses Protein in Geweben, Gewebehomogenaten oder in Körperflüssigkeiten vorhanden ist. Das Vorhandensein dieses Proteins ermöglicht die Unterscheidung von Zellen bzw. Geweben entsprechend dem Vorkommen von CK 20 in bestimmten Geweben. So wird beispielsweise die Unterscheidung von Adenokarzinomen des Gastrointestinaltraktes, der Blase und von Merkel-Zellen der Haut von anderen Adenokarzinomen ermöglicht, ebenso wie der Nachweis der zellulären Bestimmung von Metastasen, wobei Metastasen, die möglicherweise an vollkommen unabhängigen Körperstellen gefunden werden, durch den Nachweis des CK 20-Proteins einem Primärtumor der oben genannten Bereiche zugeordnet werden können. Dabei wird es ermöglicht, durch Identifizierung des Primärtumors auf die tatsächliche Tumorquelle therapeutisch einzuwirken, die häufig äußerst schwierig oder überhaupt nicht auffindbar ist nach bisher bekannten Methoden, und damit die Überlebenschancen für den Patienten erheblich zu verbessern. Ein derartiges Verfahren zur Unterscheidung der Adenokarzinome des Gastrointestinaltraktes, der Blase und von Merkel-Zellen von anderen Adenokarzinomen oder der Nachweis der zellulären Abstammung von Metastasen durch Untersuchung auf das Vorhandensein von CK 20 im zu untersuchenden Gewebe mit Hilfe der für CK 20 spezifischen erfindungsgemäßen Antikörper ist daher ein weiterer Gegenstand der vorliegenden Erfindung.

Wiederum ein weiterer Gegenstand ist die Verwendung eines erfindungsgemäßen Standardproteinmaterials zum Nachweis von Autoantikörpern gegen CK 20 in Blut oder Serum. Autoantikörper gegen CK 20 entstehen beispielsweise bei der z.B. chemotherapeutischen Behandlung von Tumoren und können ais ein Kriterium für den Verlauf der Behandlung gewertet wercen. Weiter können solche Autoantikörper auch bei anderen Krankheiten entstehen, z.B. bei Morbus Crohn, wodurch es auch mit Hilfe des Standardproteinmaterials ermöglicht wird, hier einen Hinweis auf eine Erkrankung zu erhärten oder zu widerlegen.

Außerdem kann das Standardproteinmaterial, wie bereits erwähnt, in immunologischen Tests unter Anwendung des erfindungsgemäßen Antikörpers zum Nachweis von CK 20 eingesetzt werden, wenn beispielsweise ein Immunoassay nach dem Verdrängungsprinzip durchgeführt wird.

Die mit den erfindungsgemäßen Antikörpern oder dem Standardproteinmaterial durchführbaren Immunoassays sind dem Fachmann bekannt und brauchen hier nicht weiter beschrieben werden. Es ist hierbei selbstverständlich, daß der Nachweis über eine wie auch immer geartete Markierung von Antikörper oder Standardproteinmaterial geführt wird, wobei alle bekannten tatsächlichen Testdurchführungen hier möglich erscheinen.

Auch ein Nachweis von Zelläsionen, wie es beispielsweise in der EP-A-0 057 043 beschrieben ist, kann in entsprechender Form mit dem erfindungsgemäßen Antikörper durchgeführt werden. Ebenso kann der erfindungsgemäße Antikörper eingesetzt werden, um ein Verfahren durchzuführen, wie es in der EP-A-0 057 076 beschrieben ist, analog für das Cytokeratin 20. Hierbei wird der erfindungsgemäße Antikörper als der eindeutig immunologisch reagierende Antikörper eingesetzt.

Die folgenden Beispiele sollen in Verbindung mit der Abbildung die Erfindung weiter erläutern.

Fig. 1 zeigt hierbei die partielle Aminosäuresequenz von CH 20-Fragmenten, die durch Verdauung mit Staphylococcus V8 Protease und Auftrennung durch Umkehrphasen-HPLC erhalten wurden. Diese Sequenzen werden verglichen mit den entsprechenden Sequenzen verschiedener menschlicher Typ I-Cytokeratine. Identische Aminosäuren sind hierbei fett gedruckt. Punkte bedeuten Auslassungen, die gemacht wurden, um besser Korrelationen zu zeigen.

### Beispiel 1

### Herstellung einer Cytoskelett-Präparation

In größerem Maßstab wurde eine Cytoskelettfraktion aus Duodenalschleimhautzotten präpariert. Das tiefgefrorene Zottenmaterial wurde aufgetaut, mittels eines Polytron-Homogenisators (Kinematica, Luzern, Schweiz) homogenisiert und mit einem fünffachen Volumen von Puffer A (1,5 mol/l KCl, 0,5 % Triton-X-100, 5 mmol/l EDTA, 0,4 mmol/l Phenylmethylsulfonyl-Fluorid (PMSF), 10 mmol/l Tris-HCl, pH 7,2) für 20 Minuten unter Rühren in Eis extrahiert. Durch Zentrifugation (10 Minuten bei 13000 g, 4°C) und zweifaches Waschen des Sediments (Resuspension mittels eines Dounce-Homogenisators und erneute Zentrifugation) in Puffer B (5 mmol/l EDTA, 0,4 mmol/l PMSF, 10 mmol/l Tris-HCl, pH 7,2) wurde ein Cytoskelett-Pellet ernalten, das bei -80°C aufbewahrt wurde. In analoger Weise wurden auch Cytoskelettfraktionen aus anderen Geweben und Tumoren präpariert; in diesen Fällen wurde das Gewebe vor der Polytron-Homogenisation mit einer Schere oder einem Skalpell in feine Stücke zerschnitten.

Cytoskelettfraktionen von Kulturzellen wurden nach einem ähnlichen Schema gewonnen. Die auf dem Boden von Plastikkulturflaschen adhärent gewachsenen Zellen wurden nach Abgießen des Kulturmediums und zweimaligem Abspülen mit Phosphat-gepufferter Salzlösung (PBS) mit einem Gummispatel abgeschabt und in Puffer A extrahiert, darauf mit Puffer B gewaschen (siehe Achtstätter et al., Methods Enzymol. 134 (1986) 355-371).

### Kulturzellen

Die folgenden etablierten, von menschlichen Karzinomen abstammenden Zellkulturlinien wurden bei den Untersuchungen verwendet:
1. Die Blasenkarzinom-Zellinien RT-112, RT-4, T-24 und EJ (siehe Moll et al., Am.J.Pathol. 132 (1988) 123-144).
2. Mehrere von der American Type Culture Collection (ATCC) bezogene und wie dort angegeben kultivierte Kolonkarzinom-Zellinien: HT-29 (ATCC HTB 38; LoVo (ATCC CCL 229; SW 1116 (ATCC CCL 233); LDL-1 (ATCC CCL 221); COLO 320 DM (ATCC CCL 220).
3. Zellen der menschlichen Mammakrazinom-Zellinie MCF-7 wurden wie bei Moll und Mitarb., Cell 31 (1982) 11-24) beschrieben gezüchtet. In einigen Experimenten wurden Kulturzellen metabolisch mit ³⁵S-Methionin markiert (Franke und Mitarb., Knapp und Franke, Cell 59 (1989), 67-79. "Spontaneous losses of control of cytokeratin gene expression in transformed, non-epithelial human cells occurring at different levels of regulation").

### Beispiel 2

### Präparation von reinem CK 20

Zur Gewinnung von CK 20-Proteinen wurden zwei verschiedene Proteinisolationsverfahren angewandt. Zum einen wurde die präparative Gelelektrophorese verwendet (Achtstätter und Mitarb., 1986, siehe Beispiel 1), wobei aus Duodenalschleimhaut-zotten gewonnene Cytoskelett-Proteine in eindimensionaler Elektrophorese (SDS-PAGE) aufgetrennt und durch Natriumacetat-Färbung sichtbar gemacht wurden. CK 20 wandert dabei als eine Bande mit einem Molekulargewicht von ca. 46.000. Die Proteinelution aus den ausgeschnittenen Banden erfolgte entweder elektrophoretisch oder durch Inkubation des fein homogenisierten Gelmaterials in einer 0,05 %igen wäßrigen SDS-Lösung. Das eluierte Protein wurde mittels Vakuumdialyse eingeengt und mit Aceton gefällt. Als SDS-PAGE-System wurde entweder das Laemmli-System (Laemmli, U.K., Nature 227 (1970), 680-685) oder das Puffersystem mit erhöhter Salzkonzentration (Achtstaetter und Mitarb., 1986 (s.o.)) angewendet, in manchen Fällen auch beide sukzessiv.

Als zweite Methode wurde aus gleichem Ausgangsmaterial eine Kombination von DEAE-Zellulose-Anionen-Austauscherchromatographie und "Reverse-phase"-HPLC-Chromatographie verwendet (Achtstaetter und Mitarb., 1986 s.o.). Die Reinheit der Proteinfraktionen wurde durch SDS-PAGE überprüft. Die chromatographische Methode vermeidet eine SDS-Denaturierung und war daher die Methode der Wahl für in vitro-Komplexbildungs- und Rekonstitutionsversuche.

### Beispiel 3

### Herstellung spezifischer polyklonaler Antikörper gegen CK 20

Durch präparative Gelelektrophorese gereinigtes CK 20-Protein wurde zur Immunisierung von Mäusen und Meerschweinchen eingesetzt, wobei das Immunisierungsschema von Franke und Mitarbeitern (Franke, Denk, Kalt and Schmid (1981), Biochemical and immunological identification of cytokeratin proteins in hepatocytes of mammalian liver-tissues. Exp. Cell Res. 131, 299-318) zur Anwendung kam. Es konnte ein Antiserum isoliert werden, das hohe Titer von Antikörpern gegen CK 20-Protein, jedoch auch Antikörper gegen CK 18 enthielt. Aus diesem Serum konnten monospezifische Antikörper gegen CK 20-Protein (ohne Reaktivität mit CK 18) isoliert werden, indem das in PBS mit 1 % Rinderserumalbumin und 0,1 % Natriumazid verdünnte Serum (oder eine daraus durch Ammoniumsulfat-Präzipitation hergestellte Immunglobulinfraktion) mehrfach an Nitrozellulosestreifen absorbiert wurde, an welche durch SDS-PAGE aufgetrennte und elektrotransferierte Cytokeratine 8, 18 und 19 aus MCF-7-Zellen oder MCF-7-Zell-Gesamtprotein gebunden worden waren. Die Absorption erfolgte jeweils durch 30-minütige Inkubation in einem Foliensäckchen unter ständigem Drehen. Zwischen den Absorptionsschritten wurden die Nitrozellulosestreifen durch Inkubation in 3 mol/l KSCN in PBS und anschließendes Waschen in PBS regeneriert. Es wurden sukzessive etwa vier bis acht Absorptionsschritte durchgeführt. In manchen Versuchen wurde vor dieser Gegenabsorption ein positiver Affinitätsreinigungs-Schritt an Nitrozellulose-Streifen mit SDS-PAGE-getrenntem CK 20-Protein durchgeführt; dabei wurden die an diese Streifen gebundenen Antikörper mittels 3 mol/l KSCN in PBS eluiert und gegen PBS vakuumdialysiert (Krohne und Mitarb., (J.Cell Biol. 94 (1982) 749-754). Die Spezifität der gereinigten Antikörperpräparationen wurde durch Immunblot-Analysen nach zweidimensionaler gelelektrophoretischer Auftrennung gesichert.

### Beispiel 4

### In vitro-Rekonstitution von heterotypischen Cytokeratinkomplexen und Intermediärfilamenten

Aus Cytoskelettmaterial von Duodenalschleimhaut-Zotten gewonnene, chromatographisch gereinigte Proteine (CK 8, 18 und 20) wurden in einem 10 mmol/l Tris-HCl-Puffer (pH 8,0), der 2,5 mmol/l DTT und 9,5 mol/l Harnstoff enthielt, gelöst und (nach Abzentrifugation unlöslichen Restmaterials bei 13000 g) entweder allein oder in bestimmten, annähernd stöchiometrischen Mischungen (CK 8 + CK 18; CK 8 + CK 20) gegen denselben Puffer mit DTT, aber nur 4 mol/l Harnstoff dialysiert. Dadurch sollte eine heterotypische Komplexbildung zwiscnen Typ I- und Typ II-Cytokeratinen ermöglicht werden. Aliquots der auf 4 mol/l Harnstoff gebrachten Lösung wurden (nach Zentrifugation) direkt als Proben einer Elektropnorese unter nichtdissoziierenden Bedingungen in 4 mol/l Harnstoff unterzogen, kombiniert mit SDS-PAGE in der zweiten Dimension.

Zur in vitro-Rekonstitution von Intermediär-(Cytokeratin)-Filamenten wurde chromatographisch gereinigtes CK 8 und CK 20-Protein jeweils in einer Konzentration von 1 mg/ml (Proteinbestimmung nach Bradford (Bradford M.M., Anal.Biochem. 72 (1976), 248-254) gelöst (Puffer siehe oben) und im equimolaren Verhältnis gemischt. Diese Mischung (und als Kontrollen auch Lösungen der einzelnen Proteine) wurden auf schwimmenden Membranfiltern (Millipore VS 0,025) gegen 4 mol/l Harnstoff in 10 mmol/l Tris-HCl (pH 7,6) mit 2,5 mmol/l DTT für eine Stunde dialysiert, anschließend für zwei Stunden gegen
10 mmol Tris-HCl (pH 7,6) mit 2,5 mmol/l DTT, aber ohne Harnstoff. Anschließend wurde eine Negativfärbung und elektronenmikroskopische Untersuchung durchgeführt (Quinlan et al., J.Mol.Biol. 178 (1984), 365-388).

### Beispiel 5

### Proteolytische Spaltungsexperimente

Natives Cytoskelettmaterial aus Duodenalschleimhautzotten wurde einer partiellen proteolytischen (chymotryptischen) Spaltung unterzogen (Hatzfeld und Mitarb., J.Mol.Biol. 197 (1987)237-255). Es wurden dabei Enzym Substrat-Verhältnisse von 6,6:1000 oder 9:1000 und Verdauungszeiten zwischen 30 und 60 Minuten angewendet. Die Spaltprodukte wurden mittels SDS-PAGE mit anschließender Silberfärbung oder Immunblot-Analyse oder mittels zweidimensionaler Gelelektrophorese, verbunden mit tryptischer Peptid-Kartierung, analysiert.

### Beispiel 6

### Immuncytochemie, verwendete Verfahren für die Immunfluoreszenz,

Immunperoxidase und Immunelektronenmikroskopie von kryostatischen Gewebesektionen und Kulturzellen wurden wie beschrieben angewandt (Bader et al., Eur.J.Cell Biol. 47 (1988) 300-319; Franke et al., Proc.Natl.Acad.Sci. USA 75, (1978) 5034-5038; Franke et al., Exp.Cell Res. 116 (1978) 429-445; Franke et al., Eur.J.Cell Biol. 19 (1979) 255-268; Franke et al., Exp.Cell Res. 131 (1981) 299-318; Franke et al., J.Cell Biol. 90 (1981) 116-127; Franke et al., Cell 30 (1982) 103-113; Franke et al., Virchows's Archiv A, Pathol.Anat. 411 (1987) 137-147; Jahn et al., Differentiation 36 (1987) 234-254; Knapp und Franke, Cell 59, (1989) 67-79, Moll et al., Am.J.Pathol. 132 (1988) 123-144.)

### Beispiel 7

### Verfahren zur Gewinnung eines Standards

Die angewendeten Methoden werden am Beispiel der Cytokeratine beschrieben; die Art der Aufarbeitung kann jedoch auf alle IF-Proteine übertragen werden.

### 7.1 Reinigung der intakten Polypeptide

Humancytokeratine (z.B. 8) wurden im wesentlichen wie bei Achtstaetter et al., Methods Enzymol. 134:355-371 (1986), beschrieben, aus der Humankulturzellinie MCF-7 gewonnen. Die abgeschabte Zellschicht wird homogenisiert (im wesentlichen beschrieben bei Achtstaetter et al., supra). Einzelne Cytokeratin-Polypeptide wurden mittels Anionen-Austauscherchromatographie auf DEAE-Cellulose (DE 52; Whatman Chemical Separation Inc., Clifton, NJ, USA) in einem 8 mol/l, (für Cytokeratin 8) bzw. 9,5 mol/l (für Cytokeratin 20) Harnstoff-Puffer (8 bzw. 9,5 mol/l Harnstoff, 2,5 mmol/1 Dithioerythrit, 30 mmol/l Tris-HCl (pH 8,0)) chromatographisch gereinigt, im wesentlichen wie beschrieben bei Hatzfeld & Franke, J.Cell.Biol. 101 (1985) 1826-1841; Achtstaetter et al., 1986 (s.o.); Bader et al., EMBO J. 5 (1986) 1865-1875); Quinlan et al., J.Mol.Biol 192 (1986) 337-349. Kurz beschrieben, wurde Cytoskelettmaterial für 2 Stunden in 9,5 mol/l Harnstoff (5 mmol/l Dithioerythritol, 10 mmol/l Tris-HCl (pH 8,0)) extrahiert und der nach Zentrifugation bei 100.000 x g (g = Gravitationskonstante) erhaltene Überstandextrakt wurde gegen einen 8 bzw. 9,5 mol/l Harnstoff-Puffer dialysiert und auf eine DEAE-Cellulosesäule, die mit diesem Puffer equilibriert wurde, aufgebracht. Gebundenes Protein wurde mit einem 0 bis 100 mmol/l Guanidinium-HCl-Gradienten eluiert. Die Polypeptid-Zusammensetzung wurde durch eine SDS/Polyacrylamid-Gelelektrophorese (PAGE) kontrolliert. Die vereinigten Fraktionen wurden einer weiteren Reinigung durch eine Hochdruck-Flüssig-Chromatographie mit Phasenumkehr unterworfen, wobei 0,01 % (v/v) Trifluor-Essigsäure (TFA) (Fluka, Buchs, Schweiz) als wäßriges Lösungsmittel A, 0,07 % (v/v) TFA in Azetonitril (chromatographische Qualität, Merck Darmstadt, BRD) als organische Phase (Lösungsmittel B) und eine BioRad-RP-304-Säule mit Phasenumkehr (BioRad Laboratories, Richmond, CA, USA) verwendet wurden. Die Peakfraktionen wurden gesammelt, das Azetonitril durch Vakuumverdampfung entfernt und die Fraktionen gefriergetrocknet.

### 7.2 Rekonstituierung der gereinigten Polypeptide zu Protofilamenten und IF-Proteinen

Die gereinigten Cytokeratine wurden in Puffer, der 9,5 mol/l Harnstoff enthält, gelöst. Äquimolare Mengen an Typ I und Typ II Cytokeratin wurden bei einer Endkonzentration von etwa 0,5 mg/ml gemischt und die Protofilamente und Cytokeratinfilamente wurden erhalten, indem die Polypeptidlösung gegen Puffer niedriger Ionenstärke dialysiert wurde. (Es wurden die Puffer, die bei Hatzfeld, M. und Franke, J.Cell Biol. 101: 1826-1841 (1985) beschrieben sind, verwendet.) Die Bildung von Protofilamenten und Cytokeratinfilamenten wurde elektronenmikroskopisch durch Negativ-Kontrastierung der Probe kontrolliert (vergleiche Hatzfeld und Franke, supra).

### 7.3 Darstellung α-helikaler Cytokeratin-Mittelstücke durch limitierte Proteolyse

Der proteolytische Abbau wurde mit verschiedenen Proteasen durchgeführt. In einer typischen Präparation wurden Chymotrypsin (EC 3.4.21.1 aus Rinderpankreas, z.B. von der Fa. Sigma, München) in einem Enzym-zu-Substrat-Verhältnis (Gewicht/Gewicht) von 6,6:1000 für das Cytokeratin 8:18 Paar und im Verhältnis 9:1000 für das Cytokeratin 8:20 Paar eingesetzt. Für jede Chymotrypsin-Charge muße die Abdauzeit optimiert werden. Der proteolytische Abbau wurde durch gelelektrophoretische Analysen der Abbauprodukte kontrolliert und für den maximalen Anteil an stabförmigen Mittelstücken (Mᵣ = 38.000 - 40.000) optimiert. Das Optimum liegt bei ca. 20 min bei 30°C. Nach der entsprechenden Abdauzeit wurde die Enzymaktivität durch Zugabe von 5 mM Phenylmethylsulfonylfluorid gestoppt.

### 7.4 Reinigung der α-helikalen Mittelstücke

Die proteolytischen Mittelstücke und deren einfache Fragmente wurden entweder chromatographisch auf einer Sepharose CL6B (Pharmacia LKB, Freiburg) Säule oder direkt über Umkehrphasen-Hochleistungsflüssigkeitschromatographie getrennt, und zwar auf einer BioRad RP304-Säule mit Phasenumkehr unter Verwendung des im obigen Abschnitt 7.1 beschriebenen Lösungsmittelsystems. Zur weiteren Reinigung wurden die Hauptfraktionen mit Lösungsmittei A zur Reduzierung der Azetonitril-Konzentration auf ca. 20 % (v/v) verdünnt und dann direkt auf eine My-Bondapak C18-Säule mit Phasenumkehr (Waters Associates, Milford, MA) aufgebracht. Alle Hauptfraktionen wurden lyophilisiert und die Proben mittels 1- und 2-dimensionaler Gelelektrophorese auf Anwesenheit der α-helikalen Mittelstücke, die zum Teil einfach geteilt sind zu jeweils zwei Mittelstückfragmenten, da die Schnittstelle an einem kurzen mittleren Abschnitt, an dem die helikale Struktur unterbrochen ist, liegt, wurden als Referenzmaterial bzw. Standardmaterial für die Eichung des Nachweissystems und für Immunisierungen eingesetzt.

### Beispiel 8

### Auswahl und Herstellung geeigneter Antikörper

Spezifische Antikörper gegen Intermediärfilamentproteine, sowohl eigene Entwicklungen als auch kommerziell erhältliche, wurden auf ihre Immunreaktivität mit den α-helikalen Mittelstückfragmenten, wie sie als Standardmaterial nach Beispiel 7 gewonnen wurden, untersucht mit folgenden Methoden:

### 8.1. Immunblot (Western-Blot; Reaktion mit denaturiertem Antigen)

Gereinigte Fragmentproteine (z.B. Cytokeratin 8:18-, Cytokeratin 8:20- oder Vimentin-Fragmente) und Cytoskelett-Präparationen aus Gewebeproben (z.B. Lymphknoten oder Leber) vor und nach proteolytischer Verdaureaktion mit Chymotrypsin (vergleiche Beispiel 9) wurden gelelektrophoretisch (Natriumdodecylsulfat-Polyacrylamid-Gelelektrophorese) aufgetrennt, die Proteine elektrophoretisch auf Nitrozellulose transferiert und mit den in Frage kommenden spezifischen Antikörpern inkubiert. Die Immunreaktion wurde über markiertes Protein A oder markierte Anti-Maus-Antikörper nachgewiesen. Antikörper, die mit den α-helikalen Mittelstücken (Mᵣ 38.000-40.000; Mᵣ 20.0000-22.000 im Falle der basischen Keratine) eine Immunreaktion zeigten, wurden ausgewählt.

### 3.2 Dot-Blot (Reaktion mit nativem bzw. renaturiertem Antigen)

Ca. 2 x 10⁻⁶ g gereinigte CK 20-Proteine (gelöst in 50 x 10⁻⁶ l 50 mmol/l Na₂HPO₄-Puffer, pH 7,4) bzw. Überstandsfraktionen von homogenisierten Gewebeproben nach Chymotrypsinverdau werden direkt an Nitrozellulose gebunden (z.B. in einer SRC 96 Minifold I Dot-Blot-Apparatur von Schleicher und Schuell, Kassel, BRD) und mit den in Frage kommenden, spezifischen Antikörpern inkubiert. Der weitere Arbeitsgang ist wie unter 1. beschrieben.

### 8.3 ELISA (Reaktion mit nativem bzw. renaturiertem Antigen)

500 ng (10⁻⁹ g) gerenige Fragmentproteine (gelöst in 100 µl (10⁻⁶ l) 50 mmol/l NaHCO₃-Puffer, pH 9,6) bzw. 2 µg (10⁻⁶ g) (in 100 µl [10⁻⁶l]) Protein der Überstandsfraktionen von homogenisierten Gewebeproben nach Chymotrypsinverdau werden pro Vertiefung in einer 96-Loch Mikrotiterplatte inkubiert und gebundenes Protein mit den in Frage kommenden spezifischen Antikörpern inkubiert. Der weitere Arbeitsgang ist wie unter 1. beschrieben.

### 8.4 Immunfluoreszenz-Mikroskopie. Standardmethode, eingehend beschrieben z.B. bei Ciocca D.R. und Bjercke R.J. (1986) in Methods Enzymol. 121, 562-579.

Antikörper und Antiseren, die nach den o.g. Methoden positiv waren, sind Kₛ 19.2; Kₛ 18-9B1; Kₛ 18-27 IV; Kₛ 8-17.2; Ks pan 1-8; VIM 3B4; IT-Meerschweinchen Antiserum (Herstellung von Meerschweinchen Antiserum gegen Cytokeratin 20 (siehe Beispiel 3, IT-Maus Antiserum (Herstellung s. Beispiel 3).

### 8.5 Herstellung monoklonaler, gegen α-helikale Mittelstücke gerichtete Antikörper

Zur Herstellung spezifischer Antikörper wurden nur in vitro rekonstituierte Filamente, die aus den jeweiligen gereinigter Polypeptiden gebildet wurden, zur Immunisierung injiziert. Um monoklonale Antikörper herzustellen, wurden weibliche, 6-8 Wochen alte BALB/c Mäuse immunisiert, indem ihnen Cytoskelett-Präparationen oder rekonstituierte Filamente mit 30-300 x 10⁻⁶ g Protein pro Injektion injiziert wurden. Die Antigene wurden in Phosphat-gepufferter Kochsalzlösung (PBS) suspendiert und für die erste Injektion mit Freund'schem Adjuvans (komplett) emulgiert. Bei allen folgenden Injektionen wurde Freund'sches Adjuvans (inkomplett) verwendet. Die Tiere wurden dreimal im Abstand von etwa drei Wochen subcutan gespritzt, und sie erhielten drei Tage vor der Zellfusion eine intraperitoneale Wiederholungs-Injektion mit 30-80 x 10⁻⁶ g Antigen. Die Milzzellen immunisierter Mäuse wurden mit Maus-Myelomzellen der Linie Sp3/0Ag14, X63-Ag8.653 und NSO/U (beschrieben bei Shulmann et al., Nature 276: 269-270 (1978); Kearney et al., J. Immunol. 123: 1548-1550 (1979); Clark und Milstein, Somatic Cell Genetics 7: 657-666 (1981)) im Verhältnis 10:1 fusionsiert, im wesentlichen wie es bei Koehler und Milstein, Nature 256: 495-497 (1975) beschrieben wurde. Die Hybridomüberstände wurden immunfluoreszenzmikroskopisch auf Gefrierschnitten von Human- und Rindergwebe (im wesentlichen beschrieben bei Achtstaetter et al., Differentiation 31: 206-227 (1986)) oder auf Kulturzellen, die auf speziell beschichteten Objektträgern oder Deckgläschen gezüchtet wurden, getestet oder mit der Enzym-gekoppelten Immunadsorptionstechnik (ELISA), wobei die gereinigten Antigene zum Beschichten von Mikrotiterplatten eingesetzt wurden. Positive Klone wurden zweimai duch kontrollierte Ausverdünnung subkloniert. Ig-Subklassen wurden nach Ouchterlony und Nilsson, L.A. (1978, in: Handbook of Experimental Immunology; Wei ed., vol. 1, chapter 19, Blackwell Scientific Publications, Oxford, pp. 1-19) bestimmt.

### 8.6 Koppeln der Detektor-Antikörper an Peroxidase.

Die als Detektor-Antikörper bezeichneten monoklonalen Antikörper und spezifisches Meerschweinchen-Antiserum wurden nach einem von B. Tijssen (Laboratory techniques in biochemistry and molecular biology, vol. 15: Practice and theory of enzyme immunoassays, R.H. Burdon and P.H. van Knippenberg, eds., Elsevier Amsterdam, New York, Oxford; S. 238) beschriebenen Verfahren an Peroxiaase gekoppelt:

5 mg Peroxidase werden in 0,5 ml Nateriumcarbonatpuffer (100 mmol/l, pH 9,2) gelöst und zum Koppeln vorbereitet, indem das Enzym 2 Stunden bei Raumtemperatur und unter Lichtabschluß mit 0,5 ml einer 10 mmol/l NaIO₄-Lösung oxidiert wird. Danach wird der gewünschte Antikörper (10 mg gelöst in 2 ml 100 mmol/l Natriumcarbonatpuffer, pH 9,2) zugesetzt, 0,5 g trockenes Sephadex G-25 (Fa. Pharmacia, Freibrug) zugegeben und weitere 3 Stunden unter Lichtabschluß inkubiert. Das dabei entstandene Konjugat wird aus dem Sephadex-Material mit dem Natriumcarbonatpuffer eluiert und mit 1/20 Volumteil einer 0,5 % NaBH₄-Lösung in 0,1 mmol/l NaOH gemischt. 30 min. später wird 1/10 Volumteil der gleichen Lösung zugesetzt und 1 Stunde bei 4°C inkubiert. Das Konjugat wird unter Vakuumdialyse gegen PBS dialysiert und auf ca. 0,5 ml konzentriert und anschließend auf einer Sephadex-G-200 (Fa. Pharmacia) Säule (1,0 x 50 cm) fraktioniert. Die Fraktionen (ca. 0,5 ml Volumen) werden auf ihren Anteil an Enzymaktivität und Antikörper getestet und die Fraktionen, die gleichzeitig hohe Ig-Konzentration und hohe Enzymaktivität aufweisen, zusammengefaßt.

### Beispiel 9

### Nachweis und Bestimmung von Metastasen in Lymphgewebe

### 9.1 Solubilisierung von CK 20-Proteinen, vorzugsweise Derer α-helikale Mittelstücke

Es wird zunächst das Feuchtgewicht des Lymphknotengewebes ermittelt. Das Gewebe wird im dreifachen Volumen - bezogen auf das Feuchtgewicht - einer phosphatgepufferten Kochsalzlösung (PBS) (10 mmol/l Natriumphosphat pH 7,4, 150 mmol/l Natriumchlorid) mit Hilfe eines Messerhomogenisators bis zur breiartigen Konsistenz zerkleinert (es wird der Einsatz eines Polytron-Homogenisators der Firma Kinematica, Luzern/Schweiz empfohlen). Das Homogenat wird mit Chymotrypsin inkubiert.

Zu diesem Zweck wird Chymotrypsin verwendet, das zuvor an eine Matrix (CNBr-aktivierte Sepharose 4B, Fa. Pharmacia, Freiburg) gebunden worden ist: 1 g CNBr-aktivierte Sepharose 4B werden 15 min in 1 mmol/l HCl gequollen (Gelvolumen ca. 3,5 ml/g) und mit insgesamt 200 ml 1 mmol/l HCl gewaschen. Die Salzsäurelösung wird abgesaugt und das Matrixmaterial mit 5 ml Kopplungspuffer (0,5 mol/l NaCl, 0,1 mol/l NaHCO₃, pH 8,0) gewaschen. 10 mg Chymotrypsin werden in 5 ml Kopplungspuffer gelöst und mit dem Matrixmaterial in Kopplungspuffer 2 Stunden bei Raumtemperatur unter ständiger Bewegung inkubiert. Verbliebene, nicht abgesättigte Kopplungsstellen werden danach durch Zusatz von 5 ml 0,2 mol/l Glycinlösung (pH 8,0) blockiert. Anschließend wird das Gelmaterial mit einem Überschuß an Kopplungspuffer (ca. 50 ml) und 10 ml Acetatpuffer (0,5 mol/l NaCl, 0,1 mol/l Natriumacetat, pH 4,0) gewaschen. Unter diesen Bedingungen werden ca. 60 % des eingesetzten Chymotrypsins gebunden, d.h. das Sepharose 4B-Gel enthält 1,7 mg/ml gekoppeltes Chymotrypsin. Zur besseren Handhabung wird das Gel zweifach in PBS verdünnt (Chymotrypsin-Konzentration 0,85 mg/ml).

Dem Gewebebrei wird Chymotrypsin (EC 3.4.21.1 aus Rinderpankreas, z.B. von der Fa. Sigma, München) im Verhältnis 1:1000 (berechnet auf das Feuchtgewicht des Gewebes) zugesetzt. Es folgt ein Inkubationsschritt bei 30°C (Vorzugsweise im Thermoblock, eventuell im Wasserbad). Durch Einstellen des Homogenats in Eis für 5 min wird die Abdaureaktion nach 30 min gestoppt. Das Homogenat wird 30 min bei 2 x 10⁴ g zentrifugiert und der Zentrifugationsüberstand sofort abgenommen; das gekoppelte Chymotrypsin befindet sich im Sediment.

Unter diesen Bedingungen werden 80 bis 95 Gew.-% des Materials der α-helikalen Mittelstücke in noch identifizierbarem Zustand aus den CK 20-Proteinen in die Überstandsfraktion freigesetzt und einige intakte CK 20-Proteine befinden sich ebenfalls in einem löslichen Stadium.

### 9.2 Ermittlung und Bestimmung des Vimentingehaltes

Im zentrifugierten Überstand wird Vimentin immunologisch durch einen Sandwich-ELISA bestimmt. Hierzu dient ein erstes Anti-Serum, GP-8, als Fänger-Antikörper, die gegen das α-helikale Mittelstück gerichtet sind, und ein zweiter monoklonaler Antikörper VIM-3B4 als Detektor-Antikörper, der gegen ein anderes Epitop des α-helikalen Mittelstückes, das von den ersten Epitopen unabhängig und verschieden ist, gerichtet ist. Der Fänger Antikörper (GP 8) wird in einer Konzentration von 10 x 10⁻⁶ g/ml, gelöst in 50 mmol/l NaHCO₃ (pH 9,6), auf Mikrotiterplatten beschichtet (150 x 10⁻⁶ l pro Vertiefung). Für die Standardkurve wird gereinigtes Vimentin-Fragment in Konzentrationen von 10 ng/ml bis 500 ng/ml (gelöst in Puffer: 150 mmol/l NaCl, 10 mmol/l Na₂HPO₄, pH 7,4, 0,05 % Tween 20) verwendet. Zur Messung der Vimentin-Konzentration im zentrifugierten Überstand wird diser 1:100 bis 1:500 mit dem letztgenannten Puffer verdünnt. Der mit Peroxidase markierte Detektor-Antikörper VIM 3B4 wird mit Puffer (150 mmol/l NaCl, 10 mmol/l Na₂HPO₄, pH 7,4, 1 % Rinderserumalbumin, 0,05 % Tween 20) auf eine Konzentration von 0,5 x 10⁻⁶ g/ml verdünnt und mit 150 x 10⁻⁶ l pro Vertiefung eingesetzt. Als Substrat wird o-Phenylendiamin oder ABTS (2,2'-Azino-di-[3-ethylbenzthiazolinsulfonat (6)]) verwendet. Der so erhaltene Vimentinwert dient dann als Bezugsgröße für die quantiative Auswertung der weiteren Meßergebnisse.

### 9.3 Bestimmung der Cytokeratine und Ermittlung der Cytokeratingehalte

Im zentrifugierten Überstand werden die vorhandenen Cytokeratine immunologisch durch einen Sandwich-ELISA bestimmt. Hierzu dient ein erster monoklonaler Antikörper Kₛ pan 1-8, der sogenannte Fänger-Antikörper, der gegen ein erstes, für die Cytokeratine 1 bis 8 typisches Epitop gerichtet ist. Der Fänger-Antikörper Kₛ pan 1-8 wird in einer Konzentration von 2 x 10⁻⁶ g/ml, gelöst in 50 mmol/l NaHCO₃ (pH 9,6), auf Mikrotiterplatten beschichtet (150 x 10⁻⁶ l pro Vertiefung). Für die Standardkurve werden z.B. die gereinigten Cytokeratin-Fragmente in den Kombinationen Cytokeratin 8:18 und 8:20 in Konzentrationen von 5 ng/ml bis 500 ng/ml (gelöst in Puffer: 150 mmol/l NaCl, 100 mmol/l Na₂HPO₄ pH 7,4, 0,05 % Tween 20) verwendet. Als Peroxidase-gekoppelte Detektor-Antikörper werden z.B. Kₛ 18-27 IV und Kₛ18-9B1 (für Cytokeratin 18 Fragmente) und IT-Meerschweinchen Antiserum (für Cytokeratin 20 Fragmente) eingesetzt. Zur Messung der Cytokeratin-Konzentration im zentrifugierten Überstand wird dieser 1:100 mit Puffer (150 mmol/l NaCl, 10 mmol/l Na₂HPO₄, pH 7,4, 0,05 % Tween 20) verdünnt.

Zur Standardisierung werden bekannte Mengen des nach Beispiel 7 gewonnenen Cytokeratin-Standards dem Sandwich-ELISA unterworten. Die Enzymaktivität, die der Konzentration eines jeden standardisierten Cytokeratins entspricht, wird gegen die Konzentration aufgetragen, um eine Standard Kurve zu erhalten, von der die Konzentration unbekannter Mengen eines jeden Cytokeratins interpoliert werden kann.
Das Ausmaß von Karzinom-Metastasen kann ausgedrückt werden duch das Verhältnis des bestimmten Cytokeratins und des gemessenen Vimentins in der Gewebeprcbe.

### Beispiel 10

### Quantitative Bestimmung von Cytokeratin 8:20 im Sandwich-ELISA auf Mikrotiterplatten

### 10.1 Beschichten der Mikrotiterplatten

In jede Vertiefung werden 0,2 x 10⁻⁶ g Fänger-Antikörper Kₛ pan 1-8 (gelöst in 100 - 150 x 10⁻⁶ l 50 mmol/l Natriumcarbonatpuffer pH 9,6) pipettiert. Die Platte wird abgedeckt und über Nacht bei 4°C inkubiert

### 10.2 Waschen und Blockieren

Die überschüssige Antikörperlösung aus jeder Vertiefung wird durch Absaugen entfernt. In jede Vertiefung werden 3 x hintereinander 200 x 10⁻⁶ l Waschpuffer (PBS-Tween: 150 mmol/l NaCl, 10 mmol/l Natriumphosphatpuffer pH 7,4, 0,05 % Tween 20) pipettiert und durch Umdrehen der Platte entfernt. Restfeuchtigkeit wird durch leichtes Ausklopfen der Platte auf mehrere Lagen Papierhandtücher entfernt.

Jede Vertiefung wird mit 200 x 10⁻⁶ l Abblockpuffer (150 mmol/l NaCl, 10 mmol/l Natriumphosphatpuffer pH 7,4, 0,05 % Tween 20, 1 % Rinderserumalbumin, 5 % Saccharcse; bei längerer Aufbewahrungszeit werden außerdem 0,01 % Thimerosal zugefügt) gefüllt und mindestens 1 Stunde bei Raumtemperatur inkubiert.

### 10.3 Inkubieren mit Antigen bzw. Serumproben

Standardprotein des Cytokeratin 8:20, gewonnen nach Beispiel 7 in Konzentrationen zwischen 5 ng/ml und 500 ng/ml (abhängig vom jeweiligen Detektor-Antikörper) wird in Kontrollserum (Monitrol von Merz & Dade oder Kontrollogen L und LU von Behring) aufgenommen. Das Kontrollserum wird in einer Verdünnung von 1:10 und 1:100 eingesetzt. Je Vertiefung werden 100 x 10⁻⁶ l Standardproteinlösung oder Serumproben (1:10 und 1:100 verdünnt) pipettiert und 90 min. bei Raumtemperatur inkubiert. Danach wird 4 x mit 200 x 10⁻⁶ l Waschpuffer (PBS-Tween, wie oben angegeben) gewaschen.

### 10.4 Inkubation mit Detektor-Antikörper

Mit Peroxidase gekoppelter Detektor-Antikörper (CK 20-Meerschweinchen-Antiserum) wird in Puffer (150 mmol/l NaCl, 10 mmol/l Natriumphosphatpuffer pH 7,4, 1 % Rinderserumalbumin) verdünnt (die optimale Konzentration liegt bei 0,2 - 0,5 U/ml), davon in jede Vertiefung der Mikrotiterplatte 100 x 10⁻⁶ l pipettiert und 90 min. bei Raumtemperatur inkubiert. Danach wird 2 x mit 200 x 10⁻⁶ l Waschpuffer (PBS-Tween, wie oben angegeben) und 4 x mit 200 µl destilliertem Wasser gewaschen.

### 10.5 Substratreaktion

Für eine Mikrotiterplatte wird 1 Substrattablette (10 mg) o-Phenylendiamin (Fa. Sigma) und 10 x 10⁻⁶ l 30 % H₂O₂ entweder in 10 ml 0,1 M Kaliumphosphatpuffer (pH 6,0) oder in Citrat-Phosphat-Puffer (0,0347 mol/l Citronensäure, 0,0667 mol/l di-Natriumhydrogenphosphat; pH 5,0) gelöst (bei Verwendung von Citrat-Phosphat-Puffer erhält man höhere Absorptionen). In jede Vertiefung werden 100 x 10⁻⁶ l Substratlösung (auf Raumtemperatur temperiert) pipettiert. Die Mikrotiterplatte wird abgedeckt, um die Reaktion vor Lichteinwirkung zu schützen (mit Alufolie o.ä.) und solange inkubiert (15-30 min), bis sich eine entsprechende Farbintensität entwickelt hat.

### 10.6 Abstoppen der Enzymreaktion

Durch Zugabe von 50 x 10⁻⁶ l 12,5 %iger H₂SO₄-Lösung wird die Reaktion der Peroxidase gestoppt. Bei quantitativen Bestimmungen ist zu beachten, daß für Standardprotein und Testserum nach der gleichen Zeit abgestoppt wird.

### 10.7 Auswertung

Die Mikrotiterplatten werden bei 492 nm in einem ELISA-Photometer durchgemessen.

### Beispiel 11

In jede Vertiefung einer 96-Loch Mikrotiterplatte (z.B. M 129A, Dynatech, Plochingen) werden 10 µg Standardprotein (zu Filamenten rekonstituiertes Cytokeratin 20 und Cytokeratin 8 aus einer Stammlösung von 0,5 mg/ml in 4 mol/l Harnstoff, 10 mmol/l Tris-HCl pH 7,6, 2 mmol/l Dithioerythrit, 5 mmol/l EDTA), gelöst in 100 µl phosphatgepufferter Kochsalzlösung (PBS; 150 mmol/l NaCl, 10 mmol/l Natriumphosphat pH 7,4) pipettiert und 16 h bei Raumtemperatur (RT) inkubiert. Danach werden die Vertiefungen entleert, 1 x mit je 200 pl PBS gewaschen und nicht abgesättigte Bindungsstellen durch 1 h Inkubation bei RT mit 100 µl einer 1 % BSA-Lösung (Rinderserumalbumin, gelöst in PBS) abgeblockt. Anschließend werden die Vertiefungen 3 x mit je 200 µl Waschlösung (0,05 % Tween 20, gelöst in PBS) gewaschen, mit 100 µl Patientenserum (1:500 in PBS verdünnt) 1 h bei RT inkubiert, 3x mit je 200 µl Waschlösung gewaschen und zum Nachweis spezifischer IgM-Antikörper mit einem Peroxidase-gekoppelten Anti-Human-IgM Antiserum (Rabbit anti-human Ig, µ-chain specific; Dako P 322) sowie zum Nachweis spezifischer IgG-Antikörper mit einem Peroxidase gekoppelten Anti-Human IgG Anitserum (Rabbit anti-human Ig, gamma-chain specific; Dako P 214) 1:1000 in einer 0,1 % BSA-Lösung (in PBS) 1 h bei RT inkubiert. Nach 3 x mit je 200 µl Waschlösung und 2 x mit je 200 µl Aqua destillata Waschungen werden in jede Vertiefung 100 µl Substratlösung (10 mg o-Phenylendiamin, 10 pl H₂O₂ 30 % in 10 ml Citrat-Phosphat-Puffer: 0,0347 mol/l Citronensäure, 0,0667 mol/l di-Natriumhydrogenphosphat; pH 5,0) pipettiert. Unter Lichtausschluß wird 10-30 min (je nach Farbintensität) entwickelt, die Enzymreaktion durch Zusatz von 50 µl 12,5 % H₂SO₄-Lösung gestoppt und das umgesetzte Substrat bei 492 nm Wellenlänge mit Hilfe eines ELISA-Photometers gemessen. Zur Standardisierung werden parallel Vergleichsseren mit niedrigem, mittlerem und hohem Titer an Cytokeratin 20 Autoantikörpern gemessen.

### Beispiel 12

### Immunhistochemische Lokalisierung von CK 20 (Protein IT) in Normal- und Tumorgewebe des Menschen

Die Untersuchungsmethoden wurden entsprechend bekannten Verfahren, wie sie z.B. in Beispiel 6 zitiert sind, durchgeführt.

### 1. Normalgewebe

| Epithelien: | |
|---|---|
| Magenschleimhaut (foveoläres Epithel) | +++ |
| Dünndarmschleimhaut | +++ |
| Dickdarmschleimhaut | +++ |
| Urothel | +++ |
| Merkelzellen | +++ |
| Gallenblasenschleimhaut | + |
| Thymusretikulum | + |
| Prostata | + |
| Leber | - |
| Pankreas | - |
| Niere | - |
| Epidermis | - |
| Schweißdrüsen | - |
| Talgdrüsen | - |
| Brustdrüse | - |
| Mundspeicheldrüse | - |
| Mundschleimhaut | - |
| Oesophagus-Schleimhaut | - |
| Schilddrüse | - |
| Lunge | - |
| Mesothel | - |
| Uterus | - |
| Eileiter | - |
| Nebenhoden | - |
| Nicht-epitheliale Gewebe: Sämtliche negativ | |

### 2. Tumore

| Carcinome: | |
|---|---|
| Adenocarcinom des Colon | +++ |
| Adenocarcinom des Magens | ++ |
| Adenocarcinom der Pankreas | ++ |
| Adenocarcinom der Gallenblase | ++ |
| Urothel-Carcinom | +++ |
| Merkelzellcarcinom | +++ |
| Adenocarcinom der Lunge | -* |
| Mamma-Carcinom | - |
| Adenocarcinom des Endometriums | -* |
| Adenocarcinom des Ovars | -** |
| Adenocarcinom der Niere | - |
| Schilddrüsen-Carcinom | - |
| Plattenepithelcarcinom der Mundhöhle | -* |
| Plattenepithelcarcinom der Lunge | -* |
| Plattenepithelcarcinom der Cervix | - |
| Kleinzelliges Carcinom der Lunge | -* |
| Alle nicht-epithelialen Tumore sind negativ | - |
| + = positiv | |
| ++ = stark positiv | |
| +++ = sehr stark positiv | |
| - = negativ | |
| -* = negativ, jedoch Einzelzellen gelegentlich positiv | |
| -** = negativ, außer mucinösem Ovarialcarcinom (++) | |

Die Immunhistochemie wurde durchgeführt mit spezifischen Antikörpern gegen CK 20 (aus Meerschweinchen oder Maus) und Peroxidase-gekoppelten Sekundär-Antikörpern (anti-Maus- bzw. anti-Meerschweinchen-Ig aus der Ziege) oder Peroxidase-gekoppeltem Protein A an Kryostat-Gewebeschnitten.

Prinzipiell ist diese Methode auch - bei Verwendung der Maus-Antikörper - auf Paraffinschnitte anwendbar.

Die in der Beschreibung aufgeführten Zellinien ATCC HTB 38, ATCC CCC 229, ATCC CCC 233, ATCC CCC 221 und ATCC CCC 220 stehen in der Zelliniensammlung der American Type Culture Collection, Rockville, Maryland, USA (ATCC) jedermann frei zur Verfügung und sind im ATCC-Katalog aufgeführt.

## Patentansprüche

1. Verfahren zur Reinigung von Cytokeratin 20 (CK 20, Molekulargewicht ca. 46.000),
**dadurch gekennzeichnet**,
daß man eine Cytoskelettfraktion aus CK 20 enthaltenden Zellen herstellt, die darin enthaltenen Proteine gelelektrophoretisch oder/und chromatographisch auftrennt und das CK 20 aus dem Gel bzw. der das CK 20 enthaltenden Chromatographie-Fraktion gewinnt.

2. Verfahren nach Anspruch 1,
**dadurch gekennzeichnet,**
daß man die Cytoskelettfraktion aus Duodenalschleimhaut-zotten herstellt.

3. Verfahren nach Anspruch 1,
**dadurch gekennzeichnet,**
daß man die Cytoskelettfraktion aus Kulturzellen herstellt, die insbesondere aus Kolonkarzinomen, Blasenkarzinomen oder Magenkarzinomen abgeleitet sind.

4. Verfahren nach einem der Ansprüche 1, 2 oder 3,
**dadurch gekennzeichnet,**
daß man eine erste SDS-Polyacrylamidgelelektrophorese in einem Puffersystem mit erhöhter Salzkonzentration durchführt, die das CK 20 enthaltende Bande ausschneidet und das Protein eluiert und eine zweite SDS-Polyacrylamidgelelektrophorese in einem Puffersystem mit niedriger Salzkonzentration durchführt und aus der entsprechenden Bande im Gel das gereinigte CK 20 isoliert.

5. Verfahren nach einem der Ansprüche 1 bis 3,
**dadurch gekennzeichnet,**
daß man zur chromatographischen Auftrennung zuerst eine Anionenaustauschchromatographie und dann eine Phasenumkehr-HPLC durchführt.

6. Verfahren nach Anspruch 5,
**dadurch gekennzeichnet,**
daß man die Anionenaustauscherchromatographie an DEAE-Cellulose in Anwesenheit von Harnstoff und unter Verwendung eines Eluens mit linearem Gradienten von zwischen 0 und 100 mmol/l Guanidiniumhydrochlorid durchführt und die CK 20 enthaltenden Fraktionen in der Folge der HPLC unterwirft.

7. Verfahren nach Anspruch 6,
**dadurch gekennzeichnet,**
daß man die Fraktionen enthaltend 38 bis 50 mmol/l Guanidiniumhydrochlorid der HPLC unterwirft.

8. Standardproteinmaterial,
**dadurch gekennzeichnet,**
daß es aus einem rekonstituierten Cytokeratinkomplex, enthaltend CK 20 und ein basisches Cytokeratin aus der Gruppe der Cytokeratine 1 bis 8, oder aus den durch proteolytische Spaltung hergestellten entsprechenden α-helikalen Mittelstücken dieser Proteine besteht.

9. Standardproteinmaterial nach Anspruch 8,
**dadurch gekennzeichnet,**
daß es aus einem Komplex, enthaltend CK 20 und CK 8 bzw. deren α-helikale Mittelstücke, besteht.

10. Verfahren zur Herstellung eines Standardproteinmaterials enthaltend CK 20 und ein basisches Cytokeratin,
**dadurch gekennzeichnet,**
daß man gereinigtes CK 20 und ein gereinigtes basisches Cytokeratin aus der Gruppe der Cytokeratine 1 bis 8 im equimolaren Verhältnis gemeinsam in einem harnstoffhaltigen Puffer löst und die Mischung zuerst gegen einen Harnstoff und DTT enthaltenden Puffer, dann gegen einen Puffer ohne Harnstoff dialysiert.

11. Verfahren nach Anspruch 10,
**dadurch gekennzeichnet,**
daß man nachfolgend den gebildeten Cytokeratinkomplex proteolytisch spaltet.

12. Verfahren nach Anspruch 11,
**dadurch gekennzeichnet,**
daß man die proteolytische Spaltung mit Chymotrypsin in einem Enzym zu Substratverhältnis von 6:1000 bis 10:1000 und Verdauungszeiten zwischen 30 und 60 Minuten durchführt.

13. Verfahren nach Anspruch 10 bis 12,
**dadurch gekennzeichnet,**
daß man als basisches Cytokeratin CK 8 verwendet.

14. Verfahren nach einem der Ansprüche 10 bis 13,
**dadurch gekennzeichnet,**
daß man nach einem der Ansprüche 1 bis 7 gereinigtes CK 20 verwendet.

15. Verfahren nach einem der Ansprüche 10 bis 14,
**dadurch gekennzeichnet,**
daß man die Proteine in einem 8,5 bis 10 mol/l Harnstoff und 1,5 bis 3 mmol/l DTT enthaltenden Puffer löst und die erste Dialyse gegen einen 3,5 bis 4,5 mol/l Harnstoff und 1,5 bis 3 mmol/l DTT enthaltenden Puffer durchführt.

16. Verfahren zur Herstellung von für CK 20 spezifischen Antikörpern,
**dadurch gekennzeichnet,**
daß man zur Immunisierung gereinigtes CK 20 einsetzt und nach an sich bekannten Methoden dann polyklonale oder monoklonale Antikörper erzeugt.

17. Verfahren nach Anspruch 16,
**dadurch gekennzeichnet,**
daß man nach einem der Ansprüche 1 bis 7 gereinigtes CK 20 einsetzt.

18. Verfahren nach Anspruch 16 oder 17,
**dadurch gekennzeichnet,**
daß man bei der Herstellung von polyklonalen Antiköpern zur Isolierung von monospezifischen Antikörpern die Immunoglobulinfraktion einer Immunpräzipitation und Abtrennung von gegen andere Cytokeratine gerichteten Antikörpern oder/und einer Immunpräzipitation und Abtrennung der für CK 20 spezifischen Antikörper unterwirft.

19. Verfahren nach Anspruch 18,
**dadurch gekennzeichnet,**
daß man die Immunpräzipitation und Abtrennung der für CK 20 spezifischen Antikörper durch Inkubation der erhaltenen Immunglobulinfraktion mit einer festen Phase, an die CK 20 gekoppelt wurde, durchführt.

20. Verfahren nach Anspruch 18,
**dadurch gekennzeichnet,**
daß man die Immunpräzipitation und Abtrennung von gegen andere Cytokeratine gerichteten Antikörpern durch Inkubation der erhaltenen Immunglobulinfraktion mit einer festen Phase, an die elektrophoretisch aufgereinigte Cytokeratine 8, 18 und 19 oder Gesamtprotein aus diese enthaltenden Zellen gekoppelt wurde, durchführt.

21. Verfahren nach einem der Ansprüche 18 bis 20,
**dadurch gekennzeichnet,**
daß mehrfach Immunpräzipitationsschritte durchgeführt werden.

22. Verfahren nach Anspruch 19 oder 20,
**dadurch gekennzeichnet,**
daß man als feste Phase Nitrozellulosestreifen verwendet.

23. Verwendung von Antikörpern, die nach einem der Ansprüche 16 bis 20 hergestellt wurden, zur immunologischen Identifizierung von CK 20 oder seinem durch proteolytische Spaltung erhaltenen α-helikalen Mittelstück auf Gewebeschnitten, in Gewebehomogenaten und in Körperflüssigkeitsproben.

24. Verwendung nach Anspruch 23,
**dadurch gekennzeichnet,**
daß aus einer Gewebeprobe ein Homogenat gebildet wird, die in dem Homogenat enthaltenen Intermediärfilament-Proteine proteolytisch gespalten werden, und die daraus freigesetzten α-helikalen Mittelstücke in der löslichen Phase abgetrennt werden und mit Hilfe von Antikörpern identifiziert und quantitativ bestimmt werden.

25. Verwendung nach Anspruch 23,
**dadurch gekennzeichnet**,
daß in Proben von Körperflüssigkeiten wie Blut, Blutserum und Urin die darin enthaltenen löslichen Intermediärfilament-Proteinfragmente mit Hilfe der Antikörper immunologisch identifiziert und quantitativ bestimmt werden.

26. Verwendung eines Standardproteinmaterials nach einem der Ansprüche 8 oder 9 oder hergestellt nach einem der Ansprüche 10 bis 15 zum Nachweis von Autoantikörpern gegen CK 20 im Blut oder Serum.

27. In vitro-Verfahren zur Unterscheidung der Karzinome des Gastrointestinaltraktes, der Blase und Merkel-Zellen von anderen Tumoren, speziell anderen Karzinomen oder in vitro-Nachweis der zellulären Abstammung von Metastasen durch Untersuchung auf das Vorhandensein von CK 20 in zu untersuchendem Gewebe mit Hilfe von für CK 20 spezifischen Antikörpern.

## Claims

1. Process for the purification of cytokeratin 20 (CK 20, molecular weight ca. 46,000), characterized in that a cytoskeletal fraction is prepared from cells containing CK 20, the proteins contained therein are separated by gel electrophoresis and/or chromatography and the CK 20 is obtained from the gel or from the chromatography fraction containing the CK 20.

2. Process according to Claim 1, characterized in that the cytoskeletal fraction is prepared from duodenal mucosal villi.

3. Process according to Claim 1, characterized in that the cytoskeletal fraction is prepared from culture cells derived in particular from carcinomas of the colon, carcinomas of the bladder or carcinomas of the stomach.

4. Process according to one of Claims 1, 2 or 3, characterized in that a first SDS-polyacrylamide gel electrophoresis is carried out in a buffer system of high salt concentration, the band containing the CK 20 is cut out and the protein is eluted, and a second SDS-polyacrylamide gel electrophoresis is carried out in a buffer system of low salt concentration and the purified CK 20 is isolated from the appropriate band in the gel.

5. Process according to one of Claims 1 to 3, characterized in that the chromatographic separation is performed by carrying out firstly anion exchange chromatography and then reversed phase HPLC.

6. Process according to Claim 5, characterized in that the anion exchange chromatography is carried out on DEAE-cellulose in the presence of urea using an eluent with a linear gradient of between 0 and 100 mmol/l of guanidinium hydrochloride, and the fractions containing CK 20 are subsequently subjected to HPLC.

7. Process according to Claim 6, characterized in that the fractions containing 38 to 50 mmol/l of guanidinium hydrochloride are subjected to HPLC.

8. Standard protein material, characterized in that it consists of a reconstituted cytokeratin complex containing CK 20 and a basic cytokeratin from the group comprising cytokeratins 1 to 8, or of the corresponding α-helical intermediate fragments of these proteins, prepared by proteolytic cleavage.

9. Standard protein material according to Claim 8, characterized in that it consists of a complex containing CK 20 and CK 8 or their α-helical intermediate fragments.

10. Process for the preparation of a standard protein material containing CK 2C and a basic cytokeratin, characterized in that equimolar proportions of purified CK 20 and a purified basic cytokeratin from the group comprising cytokeratins 1 to 8 are together dissolved in a buffer containing urea, and the mixture is dialyzed firstly against a buffer containing urea and DTT and then against a buffer not containing urea.

11. Process according to Claim 10, characterized in that the cytokeratin complex formed is subsequently subjected to proteolytic cleavage.

12. Process according to Claim 11, characterized in that the proteolytic cleavage is carried out with chymotrypsin in an enzyme to substrate ratio of 6:1000 to 10:1000 and with digestion times of between 30 and 60 minutes.

13. Process according to Claims 10 to 12, characterized in that CK 8 is used as the basic cytokeratin.

14. Process according to one of Claims 10 to 13, characterized in that CK 20 purified according to one of Claims 1 to 7 is used.

15. Process according to one of Claims 10 to 14, characterized in that the proteins are dissolved in a buffer containing 8.5 to 10 mol/l of urea and 1.5 to 3 mmol/l of DTT and the first dialysis is carried out against a buffer containing 3.5 to 4.5 mol/l of urea and 1.5 to 3 mmol/l of DTT.

16. Process for the preparation of antibodies specific for CK 20, characterized in that purified CK 20 is used for immunization and polyclonal or monoclonal antibodies are then produced by methods known per se.

17. Process according to Claim 16, characterized in that CK 20 purified according to one of Claims 1 to 7 is used.

18. Process according to Claim 16 or 17, characterized in that, in the preparation of polyclonal antibodies for the isolation of monospecific antibodies, the immunoglobulin fraction is subsjected to an immunoprecipitation and the separation of antibodies directed against other cytokeratins, and/or to an immunoprecipitation and the separation of the antibodies specific for CK 20.

19. Process according to Claim 18, characterized in that the immunoprecipitation and separation of the antibodies specific for CK 20 are carried out by incubation of the resulting immunoglobulin fraction with a solid phase to which CK 20 has been coupled.

20. Process according to Claim 18, characterized in that the immunoprecipitation and separation of antibodies directed against other cytokeratins are carried out by incubation of the resulting immunoglobulin fraction with a solid phase to which electrophoretically purified cytokeratins 8, 18 and 19, or total protein from cells containing the latter, have been coupled.

21. Process according to one of Claims 18 to 20, characterized in that several immunoprecipitation steps are carried out.

22. Process according to Claim 19 or 20, characterized in that nitrocellulose strips are used as the solid phase.

23. Use of antibodies prepared according to one of Claims 16 to 20 for the immunological identification of CK 20 or its α-helical intermediate fragment obtained by proteolytic cleavage, on tissue sections, in tissue homogenates and in samples of body fluids.

24. Use according to Claim 23, characterized in that a homogenate is formed from a tissue sample, the intermediate filamentous proteins contained in the homogenate are subjected to proteolytic cleavage and the α-helical intermediate fragments liberated therefrom are separated off in the soluble phase, identified with the aid of antibodies and quantitatively determined.

25. Use according to Claim 23, characterized in that the soluble intermediate filamentous protein fragments contained in samples of body fluids like blood, blood serum and urine are immunologically identified with the aid of antibodies and quantitatively determined.

26. Use of a standard protein material according to one of Claims 8 or 9, or prepared according to one of Claims 10 to 15, for the detection of autoantibodies against CK 20 in blood or serum.

27. In vitro process for distinguishing carcinomas of the gastrointestinal tract, carcinomas of the bladder and Merkel's tactile cells from other tumours, especially other carcinomas, or in vitro detection of the cellular origin of metastases by testing for the presence of CK 20 in test tissue with the aid of antibodies specific for CK 20.

## Revendications

1. Procédé de purification de cytokératine 20 (CK 20, masse moléculaire environ 46000) caractérisé en ce que l'on prépare une fraction de cytosquelette à partir de cellules contenant CK 20, on sépare par électrophorèse en gel et/ou chromatographie les protéines qui y sont contenues et on obtient la CK 20 à partir du gel ou de la fraction chromatographique contenant CK 20.

2. Procédé selon la revendication 1 caractérisé en ce que l'on prépare la fraction de cytosquelette à partir de villosités de muqueuse duodénale.

3. Procédé selon la revendication 1 caractérisé en ce que l'on prépare la fraction de cytosquelette à partir de cellules en culture qui proviennent en particulier de cancers du côlon, de cancers de la vessie ou de cancers de l'estomac.

4. Procédé selon l'une des revendications 1, 2 et 3 caractérisé en ce que l'on réalise une première électrophorèse en gel de SDS-polyacrylamide dans un système tampon à concentration en sel augmentée, on découpe la bande contenant CK 20 et on élue la protéine et on réalise une deuxième électrophorèse en gel de SDS-polyacrylamide dans un système tampon à faible concentration en sel et on isole la CK 20 purifiée à partir de la bande correspondante dans le gel.

5. Procédé selon l'une des revendications 1 à 3 caractérisé en ce que, pour la séparation chromatographique, on réalise d'abord une chromatographie d'échange d'anions puis une CLHP en phase inverse.

6. Procédé selon la revendication 5 caractérisé en ce que l'on réalise la chromatographie d'échange d'anions sur DEAE-cellulose en présence d'urée et en utilisant un éluant à gradient linéaire de 0 à 100 mmol/l de chlorhydrate de guanidinium et on soumet ensuite les fractions contenant CK 20 à la CLHP.

7. Procédé selon la revendication 6 caractérisé en ce que l'on soumet les fractions contenant 38 à 50 mmol/l de chlorhydrate de guanidinium à la CLHP.

8. Produit protéique standard caractérisé en ce qu'il consiste en un complexe de cytokératines reconstitué contenant CK 20 et une cytokératine basique du groupe des cytokératines 1 à 8 ou en les segments moyens en hélice α correspondants de ces protéines produits par clivage protéolytique.

9. Produit protéique standard selon la revendication 8 caractérisé en ce qu'il consiste en un complexe contenant CK 20 et CK 8 ou leurs segments moyens en hélice α.

10. Procédé de préparation d'un produit protéique standard contenant CK 20 et une cytokératine basique caractérisé en ce que l'on dissout conjointement dans un tampon contenant de l'urée de la CK 20 purifiée et une cytokératine basique purifiée du groupe des cytokératines 1 à 8 dans des proportions équimolaires et on dialyse le mélange d'abord contre un tampon contenant de l'urée et du DTT puis contre un tampon sans urée.

11. Procédé selon la revendication 10 caractérisé en ce que l'on clive ensuite par protéolyse le complexe de cytokératines formé.

12. Procédé selon la revendication 11 caractérisé en ce que l'on réalise le clivage protéolytique avec la chymotrypsine dans un rapport enzyme à substrat de 6 :1000 à 10 :1000 et avec des temps de digestion compris entre 30 et 60 minutes.

13. Procédé selon les revendications 10 à 12 caractérisé en ce que l'on utilise CK 8 comme cytokératine basique.

14. Procédé selon l'une des revendications 10 à 13 caractérisé en ce que l'on utilise une CK 20 purifiée selon l'une des revendications 1 à 7.

15. Procédé selon l'une des revendications 10 à 14 caractérisé en ce que l'on dissout les protéines dans un tampon contenant 8,5 à 10 mol/l d'urée et 1,5 à 3 mmol/l de DTT et on réalise la première dialyse contre un tampon contenant 3,5 à 4,5 mol/l d'urée et 1,5 à 3 mmol/l de DTT.

16. Procédé de préparation d'anticorps spécifiques de CK 20 caractérisé en ce que l'on utilise de la CK 20 purifiée pour l'immunisation puis on produit des anticorps polyclonaux ou monoclonaux selon des méthodes connues en soi.

17. Procédé selon la revendication 16 caractérisé en ce que l'on utilise une CK 20 purifiée selon l'une des revendications 1 à 7.

18. Procédé selon la revendication 16 ou 17 caractérisé en ce que, lors de la préparation d'anticorps polyclonaux pour l'isolement d'anticorps monospécifiques, on soumet la fraction d'immunoglobulines à une immunoprécipitation et à une séparation d'anticorps dirigés contre d'autres cytokératines et/ou à une immunoprécipitation et à une séparation des anticorps spécifiques de CK 20.

19. Procédé selon la revendication 18 caractérisé en ce que l'on réalise l'immunoprécipitation et la séparation des anticorps spécifiques de CK 20 par incubation de la fraction d'immunoglobulines obtenue avec une phase solide à laquelle CK 20 a été couplée.

20. Procédé selon la revendication 18 caractérisé en ce que l'on réalise l'immunoprécipitation et la séparation d'anticorps dirigés contre d'autres cytokératines par incubation de la fraction d'immunoglobulines obtenue avec une phase solide à laquelle ont été couplées des cytokératines 8, 18 et 19 purifiées par électrophorèse ou les protéines totales provenant de cellules les contenant.

21. Procédé selon l'une des revendications 18 à 20 caractérisé en ce que des étapes d'immunoprécipitation sont réalisées plusieurs fois.

22. Procédé selon la revendication 19 ou 20 caractérisé en ce que l'on utilise des bandes de nitrocellulose comme phase solide.

23. Utilisation d'anticorps qui ont été préparés selon l'une des revendications 16 à 20 pour l'identification immunologique de CK 20 ou de son segment moyen en hélice a obtenu par clivage protéolytique sur des coupes de tissus, dans des homogénats de tissus et dans des échantillons de liquides biologiques.

24. Utilisation selon la revendication 23 caractérisée en ce qu'un homogénat est formé à partir d'un échantillon de tissu, les protéines des filaments intermédiaires contenues dans l'homogénat sont clivées par protéolyse et les segments moyens en hélice a libérés de celles-ci sont séparés dans la phase soluble et sont identifiés à l'aide d'anticorps et déterminés quantitativement.

25. Utilisation selon la revendication 23 caractérisée en ce que, dans des échantillons de liquides biologiques tels que le sang, le sérum sanguin et l'urine, les fragments solubles de protéines des filaments intermédiaires qui y sont contenus sont identifiés par voie immunologique à l'aide des anticorps et sont déterminés quantitativement.

26. Utilisation d'un produit protéique standard selon l'une des revendications 8 et 9 ou préparé selon l'une des revendications 10 à 15 pour la mise en évidence d'auto-anticorps contre CK 20 dans le sang ou le sérum.

27. Procédé in vitro pour différencier les cancers des voies gastro-intestinales, de la vessie et des mélanoblastes d'autres tumeurs, en particulier d'autres cancers ou mise en évidence in vitro de l'origine cellulaire de métastases par recherche de la présence de CK 20 dans des tissus à étudier à l'aide d'anticorps spécifiques de CK 20.
